# EUROPEAN PATENT APPLICATION

(11) **EP 0 953 322 A2**
(43) Date of publication of application: **03.11.1999**
(21) Application number: 99303354.7
(22) Date of filing: 29.04.1999
(51) Int. Cl.: A61F 5/44

(54) **Medical device, e.g. a urostomy tap, and method of manufacture.**

(30) Priority: 29.04.1998 GB 9809156
(71) Applicant: BRISTOL-MYERS SQUIBB COMPANY, Princeton, NJ 08543-4000 (US)
(72) Inventor: Barrett, Howard Michael, Pulborough, W. Sussex RH20 1BQ (GB)
(74) Representative: Holmes, Miles Keeton

(57) **Abstract**

An ostomy, incontinence or woundcare device includes first and second portions which are movable relative to each other. The device can be formed by an integral moulding process such that the first and second portions are moulded integrally in a mounted position, but without bonding therebetween.

In one form, the device is a urostomy tap (8') in which a first portion (tap casing 10') is integrally moulded around a second portion (tap member 20'). The first and second portions can be such that it would be impossible to assemble them as separate parts.

Another aspect of the invention is an in-line style tap (8') in which the tap casing (10') consists of first and second passages (12', 14') which overlap and are offset relative to each other. This can provide a compact design of tap.

## Description

This invention relates generally to a medical device, in particular, an ostomy device or an incontinence device or a woundcare device, and to a method of production. In one aspect, the invention is particularly suitable for a device which includes first and second parts which are movable relative to each other, and are mounted one within, or around, the other; however the invention is not limited exclusively to this. In another aspect, the invention relates to a tap for medical or hygiene applications.

One example of an application of the invention is the production of taps for urostomy appliances. Many different types of tap are know. A common type of urostomy tap generally comprises a first part which is rotatably mounted within a second part, the tap being operated by relative rotation between the open and closed positions. The tap parts are individually moulded from plastics material, and the parts are assembled together to form the finished tap.

It has been appreciated that such manual assembly of the tap parts is relatively expensive and labour intensive. Also, the tap parts have to be designed to be able to clip together relatively easily without damage to either part, yet still achieve a good seal to prevent the leakage of fluid when the tap is in its closed position. Similar problems are also experienced in other sealing or non-sealing devices which include moving parts, and which have to be manually assembled together.

The present invention has been devised bearing the above in mind.

In contrast to the above, one aspect of the present invention is to mould a first portion of the device, and then to mould a second portion of the device in a mounted position relative to the first portion, such that the first and second portions are movable relative to each other.

The material used for the first portion may be different from that used for the second portion. The materials may be selected so that they are incompatible bondwise (i.e. the materials do not form a chemical bond when moulded together or one after the other).

Additionally or alternatively, the moulding technique may be such that bonding between the first and second portions will not occur.

A suitable technique for moulding the device would be multi-shot moulding (for example 2-shot moulding).

The present invention can provide the following significant advantages:
(a) The method avoids having to assemble the tap or other device from distinct parts, which is a labour-intensive operation. As mentioned above, the assembly process can sometimes damage the individual device parts, leading to faulty operation or premature failure. This is particularly undesirable in medical and hygiene applications.
(b) The technique enables shapes to be used which would, for practical reasons, be very difficult or even impossible to assemble together as separate parts. Such shapes or portions are referred to herein as generally "anti-assemblable" which term is to be interpreted broadly as meaning that the shapes or portions do not lend themselves to assembly together. For example, such shapes might include interengaging profiles for holding the first portion captive to the second portion, or for limiting relative movement between the portions. The integral moulding technique can therefore provide designers with much greater design freedom and flexibility than prior art techniques.
(c) For devices which rely on a seal between the portions, the integral moulding of the two portions can enable a perfect or near perfect seal to be achieved between the two portions, even though the portions do not bond together. In other words, the confronting surfaces of the first and second portions can be moulded in close face-to-face contact, so that the chances of leakage through the device are reduced.

In a closely related aspect, the invention provides a device comprising a first portion and a second portion movable relative to the first portion, the first and second portions being integrally moulded together without bonding therebetween.

Preferably, the device is an ostomy device, or an incontinence device, or a woundcare device.

Preferably, the first portion is mounted within, or around, the second portion.

Preferably, the first portion is rotatable relative to the second portions.

Preferably, the first and second portions are such that they are generally anti-assemblable (i.e. the portions are shaped such that it would be very difficult in practice to assemble the portions together if they were to be moulded separately).

In another aspect, the invention provides an in-line-style tap comprising a tap casing having first and second passages located towards opposite ends of the tap casing, the first passage being offset relative to the second passage, and a tap member received in one of the passages, the tap member including an opening for communicating with the other passage when the tap member is rotated to an open position. Preferably the opening moves out of the communication with said other passage when the tap member is rotated to a closed position.

By employing offset passages in the tap casing, many of the problems of designing an in-line-style tap can be avoided. Such an offset tap is especially suitable for the integral moulding techniques described in the first and second aspects. However, if desired, the tap could also be produced by moulding the tap casing and the tap member as separate parts, and assembling together these parts after moulding.

Preferably, the axis of the passages are generally parallel. It may be found convenient for the walls of the passages to diverge slightly towards their open ends, in order to aid removal of moulding tools during manufacture.

Preferably, the periphery of the first passage overlaps with the periphery of the second passage.

Preferably, the passages have a generally circular interior shape.

In another aspect, the invention provides a tap which may include any combination of features described herein and/or illustrated in the drawings.

In another aspect, the invention provides a method of producing a tap, the method including any combination of features described herein.

Embodiments of the invention are now described with reference to the accompanying drawings, in which:
Fig. 1 is a sectional view through a first embodiment in the form of a tap, shown in its open condition;
Fig. 2 is a sectional view similar to Fig. 1, but showing the tap in its closed condition;
Fig. 3 is a perspective view of the tap casing (in isolation) for the tap of Figs. 1 and 2;
Fig. 4 is a view from one side of the tap casing of Fig. 3;
Fig. 5 is a sectional view along the line V-V of Fig. 4;
Fig. 6 is a sectional view along the line VI-VI of Fig. 4;
Fig. 7 is an underside perspective view of the tap member (in isolation) for the tap of Figs. 1 and 2;
Fig. 8 is a sectional view through the tap member of Fig. 7;
Fig. 9 is a sectional view (similar to Fig. 1) showing a second embodiment of the invention; and
Fig. 10 is a part cut-away view showing a third embodiment of the invention in the form of a connector.

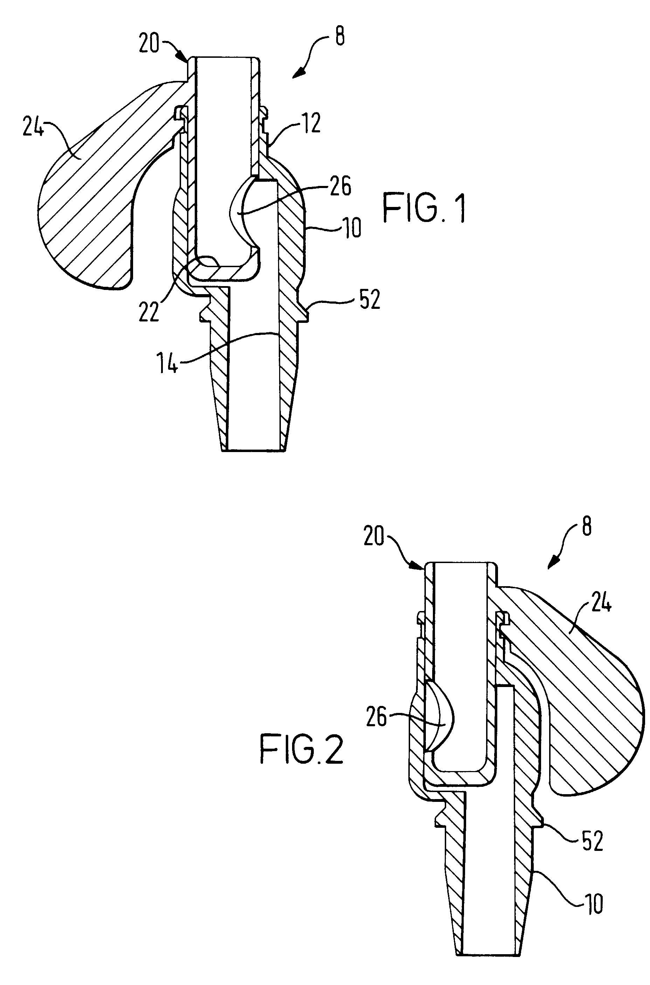

Referring to Figs. 1-6, a urostomy tap 8 comprises a hollow tap casing 10 in which is received a rotatable tap member 20. As explained in more detail below, in this embodiment, the tap casing 10 and the tap member 20 can either be integrally moulded in their mounted positions, or the members can be moulded separately and assembled together by insertion of the tap member 20 into the casing 10.

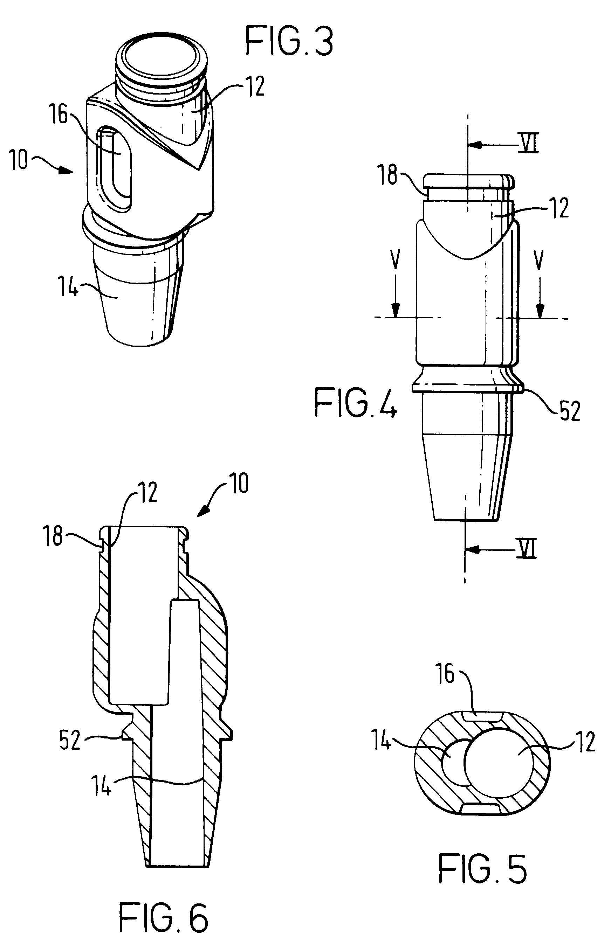

The tap casing 10 consists of offset, but overlapping, first and second passages 12 and 14 which meet in the central region of the tap casing. As can be seen in Fig. 6, the two passages form a generally figure-of-eight shape. Small recesses 16 are formed in the outer surface to aid moulding; however, as best seen in Fig. 6, the recesses do not extend all of the way through the wall of the tap casing 10.

The tap member 20 consists of a closed-ended tube 22 having a handle wing 24. An opening 26 is formed in one side of the tube 22 adjacent to the closed end.

If the tap member 20 and the tap casing 10 are moulded independently of each other, then the tap is assembled after moulding by inserting the tube 22 into one of the passages 12 and 14.

The handle 24 permits the tap member to be rotated relative to the casing. In an open position (Fig. 1), the opening 26 is aligned generally with the overlap between the passages 12 and 14, to permit fluid communication from the interior of the tube 22 to the other passage. When the tap member 20 is rotated to a closed position (Fig. 2), the opening 26 is moved out of communication with said other passage, to shut off the fluid flow through the tap.

Although the tap member 20 and the tap casing 10 can be moulded separately, it is preferred that the tap member 20 and the tap casing 10 be moulded integrally, for example using a multi-shot moulding process. In one preferred method, the tap member 20 is moulded first and the tap casing 10 is moulded in position as a second moulding stage. In an alternative method, the order of moulding can be reversed.

The plastics materials used for the tap casing 10 and for the tap member 20 can be selected so that there is no chemical bond between the tap member and the tap casing even though the two parts are moulded integrally. For example, a suitable material for the tap casing 10 is high density polyethylene (HDPE). A suitable material for the tap member 20 is nylon 6.6. Many other combinations of materials which are suitable for multi-shot moulding without chemical bonding are known to the skilled man.

The handle includes a small projection 28 which is received in an annular recess 18 of the casing 10 to prevent withdrawal of the tap member 20 from the casing 10. In this embodiment (which could be manufactured using manual assembly), the projection is profiled with a gradual lead-in ramp surface 30 to enable the projection 28 to snap into engagement with the recess 18 during such assembly, if used.

The maximally open and closed position of the tap member can be defined by interference between the handle 24 and the exterior surface of the tap casing 10.

The use of overlapping, offset passages 12 and 14 can provide a relatively straightforward design of in-line tap with a tap member which rotates about an axis aligned generally with the "in-line" direction. Generally such a type of in-line tap is difficult to design, because the fluid flow has to be at least partly lateral through the port in the tap member. Many so-called "in-line" designs incorporate a laterally extending tube, which makes the tap bulkier. In contrast, the illustrated embodiment can provide a very compact design.

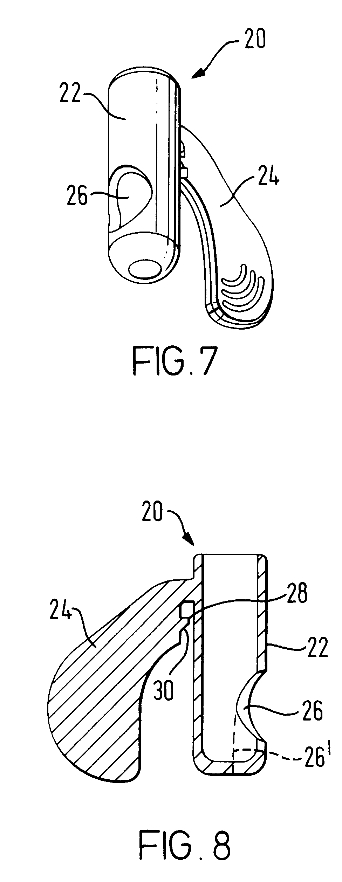

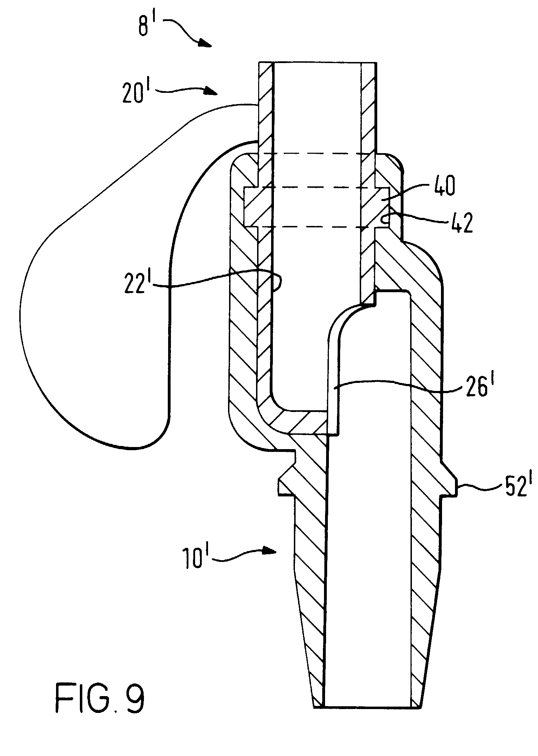

Additionally, the offset passages can provide space inside the tap casing 10 for the tap member 10 to be moulded in situ. For example, if the tap casing were to be moulded first, then additional mould parts could be inserted into the passages 12 and 14 to define the tap member 20. Any difficulties in moulding the opening 26 can be avoided by moulding the tap member 20 in its open condition (Fig. 1) which provides full access to the region of the opening 26 through the passage 14. It may be found convenient to cut back the opening as depicted by the broken line 26' in Fig. 8 (and also illustrated in the second embodiment in Fig. 9). This would allow easy extraction of the mould part in a linear direction after moulding. It is, of course, also possible to mould the tap member 20 first, and to mould the casing 10 around the tap member in a later moulding step.

Referring to Fig. 9, a second embodiment of tap 8' is illustrated. This is similar to the tap 8 of the first embodiment, and the same reference numerals, followed by a dash, are used to denote equivalent features where appropriate.

The main difference between the first and second embodiments is that the tap 8' of the second embodiment is necessarily formed by integral moulding. In other words, it would be very difficult to assemble the tap from separately moulded parts. In the second embodiment, the projection 28 on the handle is replaced by an annular rib 40 projecting from the tube 22'. The rib 40 engages in a complementary annular groove 42 in the wall of the casing 10'. The rib 40 and the groove 42 have a generally square profile to ensure that the tap member 20' is retained securely in position. However, such a square profile means that it would be practically impossible for the two parts to be assembled together as separate items.

The use of an internal rib 40 and groove 42 (or other engaging surfaces) can result in a much cleaner and neater outer surface than that obtained, for example, with the first embodiment. Moreover, the risk of the casing becoming caught or fouled on a person's clothing can be very much reduced.

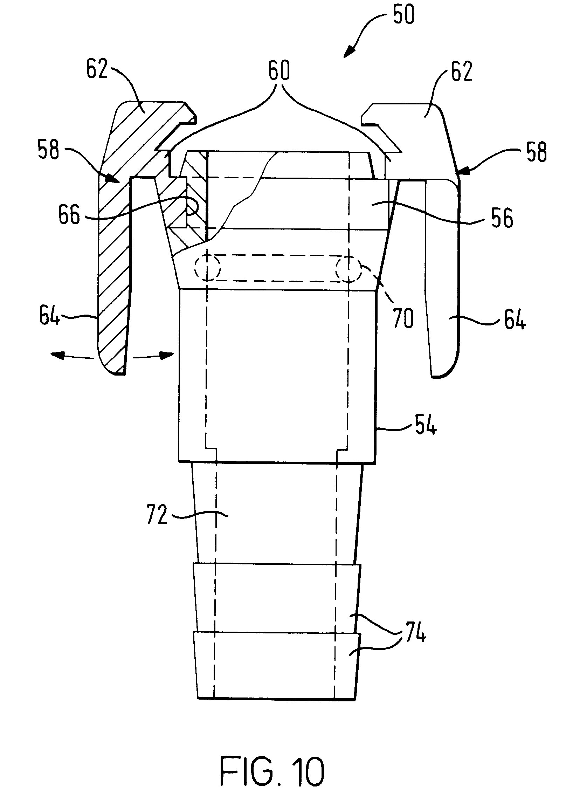

Fig. 10 illustrates a third embodiment of the invention, in the form of a connector 50. This type of connector might typically be used to couple an overnight drainage tube to the outlet of a urostomy bag. For example, the connector could mate with the annular rib 52 shown on the tap casing 10 (10') in Figs. 1-9.

The connector comprises a tubular body 54 on which is mounted a rotatable collar 56 carrying first and second latches 58. Each latch pivots about an attachment point 60 to the collar, and consists generally of a latch jaw 62 and a rearwardly extending arm 64.

In use, the jaws 62 form a snap fit over the projection 52 when the connector is advanced on to the outlet from the tap. The engagement between the latch jaw 62 and the projecting rib 52 prevents accidental removal of the connector from the tap outlet.

To disconnect the connector 50, the wearer squeezes the arms 64 inwardly, thereby moving the latch jaws 62 outwardly. In this position, the jaws 62 no longer engage the projecting rib 52, and the connector 50 can be slid off the tap outlet.

An important feature of this embodiment is that the collar 56 is rotatably mounted on the connector body 54. This enables the latches 58 to swivel about the axis of the connector, and therefore allows the wearer to position the latches so that neither latch is pressing uncomfortably against the wearer's skin. Furthermore, especially when worn at night, the wearer is likely to move while sleeping. The ability of the latches to rotate is important to allow the latches to adopt a position in which they do not bear uncomfortably against the skin if the wearer should move.

In the present embodiment, the collar 56 is received captively in an annular groove 66. Although it is possible to design the collar to be assemblable to the connector body 54, it is preferred in accordance with the principles of this invention to integrally mould the collar 56 with the connector body 54. Typically, the connector body 54 would be moulded as a first shot of a multi-shot moulding process, and the collar 56 carrying the latches 58 would be moulded as a second shot of the process. However, the moulding order may be reversed if desired.

Integral moulding avoids the additional time and cost of manually assembling the collar 56 and the connector body 54. It can also provide a significantly more secure connection between the collar 56 and the connector body. This can increase the durability of the connector, and also make the connector less prone to failure if, for example, the wearer were to lie on the connector while asleep in bed.

As shown in phantom at 70, the connector 50 may typically include an interior sealing member to form a liquid-tight seal with the tap outlet. Such a seal could be inserted manually. However, it may be more convenient and advantageous to form the sealing member integrally using integral moulding.

As can be seen in Fig. 10, the connector body 54 has a rear extension 72 carrying a plurality of barbed ridges 74. The rear extension is intended to be inserted into a drainage tube (not shown), and the barbed ridges form a tight fit with the tube material.

In this embodiment, the connector body 54 is typically made of plastics such as polypropylene. The collar 56 carrying the latches 58 may typically be made of thermoplastic elastomer. The skilled man will be aware of other suitable plastics materials which can be used in an integral moulding process without resulting in a chemical bond between the materials.

Although the connector of Fig. 10 has been described for use with the tap of Figs. 1-8 or Fig. 9, it will be appreciated that the connector may be used with any suitable complementary connector member.

The invention, particularly as described in the preferred embodiments, can provide many advantages to any suitable ostomy, wound care or incontinence device. In one aspect, integral moulding of first and second portions of a device, without bonding together, can overcome many of the problems discussed above for devices which have to assembled from separate parts. In another aspect, the use of offset, overlapping passages in an in-line tap can enable a simple and compact tap to be produced.

Different materials may be used for the first and second portions as desired. For example, in the third embodiment, the connector body 54 is made of a generally rigid plastics, and the latch collar is made of a resiliently flexible plastics. However, in the first and second embodiments, the tap casing 10 and the tap member 20 are both made of a relatively rigid material.

It will be appreciated that many modifications may be made within the scope and/or principles of the invention. Although features believed to be of importance are identified in the foregoing description and appended claims, the applicant claims protection for any novel feature or idea described herein and/or illustrated in the drawings whether or not emphasis has been placed thereon.

## Claims

1. A method of producing an ostomy and/or incontinence and/or woundcare device, the method comprising moulding a first portion of the device, and moulding a second portion of the device in a mounted position relative to the first portion without chemical bonding to the first portion, such that the first and second portions are movable relative to each other.

2. A method according to claim 1, wherein the second portion is moulded at least partly around the first portion.

3. A method according to claim 1, wherein the second portion is moulded at least partly within the first portion.

4. A method according to claim 1, 2 or 3, wherein the second portion is moulded to be captive on the first portion.

5. A method according to claim 1, 2, 3, or 4, wherein the second portion is rotatably or pivotally movable relative to the first portion.

6. A method according to any preceding claim, wherein the first and second portions are moulded so as to be anti-assemblable (as defined herein).

7. A method according to any preceding claim, wherein the moulding of the first portion and the moulding of the second portion comprise respective shots of a multi shot moulding process.

8. A method according to any preceding claim, wherein the first and second portions are of non-identical plastics.

9. A method according to any preceding claim, wherein the device is a tap.

10. An ostomy and/or incontinence and/or woundcare device produced by the method of any preceding claim.

11. An ostomy and/or incontinence and/or woundcare device comprising a first portion and a second portion movable relative to the first portion, the first and second portions being integrally moulded together without bonding therebetween.

12. A device according to claim 11, wherein the second portion is at least partly around the first portion.

13. A device according to claim 11, wherein the second portion is at least partly within the first portion.

14. A device according to claim 11, 12 or 13, wherein the second portion is captive on the first portion.

15. A device according to claim 11, 12, 13, or 14, wherein the second portion is rotatably or pivotally movable relative to the first portion.

16. A device according to any of claims 11 to 15, wherein the first and second portions are anti-assemblable (as defined herein).

17. A device according to any of claims 11 to 16, wherein the first and second portions are of non-identical plastics.

18. A device according to any of claims 11 to 17, wherein the device is a tap.

19. An in-line-style tap comprising a tap casing having first and second passages located towards opposite ends of the tap casing, the first passage overlapping, and being offset relative to, the second passage, and a tap member received in one of the passages, the tap member including an opening for communicating with the other passage when the tap member is rotated to an open position.

20. A tap according to claim 19, wherein the passages are generally circular.

21. A tap according to claim 19 or 20, wherein the tap member is integrally moulded with the tap casing.

22. A tap according to claim 19, 20 or 21, wherein the tap is a tap for a urostomy appliance.

23. A urostomy appliance comprising a tap as defined in any of claims 18 to 22.
